# EUROPÄISCHE PATENTSCHRIFT

(11) **EP 4 574 114 B1**
(45) Veröffentlichungstag und Bekanntmachung des Hinweises auf die Patenterteilung: **30.09.2026**
(21) Anmeldenummer: 23218756.7
(22) Anmeldetag: 20.12.2023
(51) Int. Cl.: A61F 13/0203, A61F 13/0206, A61F 13/0246

(54) **INSELVERBAND FÜR EINE WUNDBEHANDLUNG MIT EINEM ACRYLATKLEBSTOFF IM RANDBEREICH UND EINEM SILIKONGEL IM WUNDKONTAKTIERENDEN BEREICH**
ISLAND DRESSING FOR WOUND TREATMENT COMPRISING AN ACRYLATE ADHESIVE IN THE EDGE REGION AND A SILICONE GEL IN THE WOUND CONTACT REGION
PANSEMENT EN ÎLOTS POUR UN TRAITEMENT DE PLAIES, COMPRENANT UN ADHÉSIF ACRYLIQUE DANS LA ZONE PÉRIPHÉRIQUE ET UN GEL SILICONE DANS LA RÉGION DE CONTACT AVEC LA PLAIE

(43) Veröffentlichungstag der Anmeldung: 25.06.2025
(73) Patentinhaber: Paul Hartmann AG, 89522 Heidenheim (DE)
(72) Erfinder: CROIZAT, Pierre, 89522 Heidenheim (DE); ECKSTEIN, Axel, 89522 Heidenheim (DE)
(74) Vertreter: Paul Hartmann AG Patents & Licensing

(56) Entgegenhaltungen:
- EP-A1- 4 205 712
- US-A1- 2014 249 495
- US-A1- 2018 125 722
- US-A1- 2021 085 819
- US-A1- 2022 062 059

## Beschreibung

Die vorliegende Erfindung betrifft einen Verband für eine Wundbehandlung. Der Verband umfasst eine Rückschicht und eine erste adhäsive Schicht, welche an einer wundzugewandten Seite der Rückschicht angeordnet ist. Weiterhin umfasst der Verband ein Wundkissen und eine Wundkontaktschicht. Dabei ist das Wundkissen zwischen der Rückschicht und der Wundkontaktschicht angeordnet. Die Rückschicht mit der ersten adhäsiven Schicht überragt sowohl das Wundkissen als auch die Wundkontaktschicht, so dass der Verband einen ersten adhäsiven Rand bestehend aus der Rückschicht und der ersten adhäsiven Schicht aufweist. Die vorliegende Erfindung betrifft auch einen Kit sowie ein System für eine Unterdruckwundtherapie mit dem zuvor beschriebenen Verband.

Im Stand der Technik sind Wundverbände mit einer perforierten, Silikonklebstoffbeschichteten Wundkontaktschicht bekannt, zum Beispiel aus der US 2018/125722 A1 oder der US 2021/085819 A1. Dabei kann die perforierte, silikonisierte Wundkontaktschicht die gesamte wundzugewandte Seite des Verbands bedecken. Der Verband wird dann mit dem Silikonklebstoff am Körper des Patienten befestigt. Derartige Verbände werden auch für die Unterdruckwundtherapie eingesetzt.

Silikonklebstoffe sind üblicherweise schwach haftend und atraumatisch ausgebildet. Sie können an trockener Haut in der Wundumgebung, normalerweise jedoch nicht an der Wundoberfläche schwach anhaften. Wundverbände des zuvor genannten Typs haben folglich den besonderen Vorteil, dass sie schmerzfrei und ohne die Wunde sowie die intakte Haut in der Wundumgebung zu beschädigen wieder von der Wundstelle entfernt werden können.

Allerdings können sich solche silikonisierten Wundverbände manchmal vorzeitig von der Wundstelle ablösen. Dies ist auf die bereits erwähnte geringe Haftstärke der Silikonklebstoffe zurückzuführen. Die geringe Haftstärke der Silikonklebstoffe kann insbesondere dann nachteilig sein, wenn der Verband im Rahmen einer Unterdruckwundtherapie verwendet wird. Denn bei dieser Form der Wundbehandlung muss der Verband luftdicht an der Wundstelle anliegen und darf keine Undichtigkeiten aufweisen. Andernfalls kann der gewünschte Unterdruck im Wundraum nicht aufgebaut werden.

Um das zuvor beschriebene Problem zu lösen, können die genannten Verbände zusätzlich an ihren Rändern mit Klebestreifen an der Wundstelle fixiert und abgedichtet werden. Dies ist beispielsweise in der WO 2011/144888 A1 beschrieben und in deren Figur 24D gezeigt. Das Anbringen der Klebestreifen ist jedoch ein zusätzlicher Arbeitsschritt für das Pflegepersonal. Es wäre wünschenswert, wenn auf sekundäre Verbandsmaterialien wie die in der WO 2011/144888 A1 vorgeschlagenen Klebestreifen verzichtet werden könnte, ohne dass sich die Verbände vorzeitig von der Wundstelle ablösen oder undicht werden. Damit könnte das Pflegepersonal entlastet und der Patient kostengünstiger behandelt werden.

Der vorliegenden Erfindung lag die Aufgabe zugrunde, einen verbesserten Verband für die Wundbehandlung, insbesondere für die Unterdruckwundtherapie, zur Verfügung zu stellen. Der Verband sollte gut an der Wundstelle haften können, damit der Verband während der Wundbehandlung möglichst keine Undichtigkeiten ausbilden und sich nicht vorzeitig von der Wundstelle ablösen kann. Auf zusätzliche sekundäre Verbandsmaterialien zur Befestigung und Abdichtung wie die zuvor erwähnten Klebestreifen sollte für den neuen Verband so weit wie möglich verzichtet werden können, damit der Verband einfacher, schneller und kostengünstiger angewendet werden kann. Gleichzeitig sollte sich der Verband von der Wundstelle auch wieder möglichst leicht entfernen lassen können, damit der Verband möglichst schmerzfrei und ohne die Wundoberfläche zu beschädigen gewechselt werden kann. Die genannten Aufgaben wurden mit einem Verband für eine Wundbehandlung nach Anspruch 1, einem Kit für eine Unterdruckwundtherapie nach Anspruch 20 sowie einem System für eine Unterdruckwundtherapie nach Anspruch 21 gelöst.

Der erfindungsgemäße Verband umfasst eine Rückschicht und eine adhäsive Schicht, welche an einer wundzugewandten Seite der Rückschicht angeordnet ist. Die adhäsive Schicht der Rückschicht wird vorliegend auch als "erste adhäsive Schicht" bezeichnet. Weiterhin umfasst der Verband ein Wundkissen und eine Wundkontaktschicht.

Das Wundkissen ist zwischen der Rückschicht und der Wundkontaktschicht angeordnet. Die Rückschicht mit der ersten adhäsiven Schicht überragt sowohl das Wundkissen als auch die Wundkontaktschicht, so dass der Verband einen adhäsiven Rand bestehend aus der Rückschicht und der ersten adhäsiven Schicht aufweist. Der adhäsive Rand des Verbands wird vorliegend auch als "erster adhäsiver Rand" bezeichnet. Der erfindungsgemäße Verband ist demnach in der Art eines Inselverbands ausgestaltet.

Der erfindungsgemäße Verband zeichnet sich durch einen besonderen Aufbau der Wundkontaktschicht und eine besondere Klebstoffkombination aus. So besteht die erste adhäsive Schicht aus einem Acrylatklebstoff mit einer Klebkraft von mehr als 4 N/25 mm wie nachfolgend noch genauer erläutert. Weiterhin besteht die Wundkontaktschicht aus einer perforierten Trägerfolie, an deren wundzugewandten sowie wundabgewandten Seite jeweils eine adhäsive Schicht angeordnet ist. Die wundzugewandte adhäsive Schicht der Trägerfolie wird vorliegend auch als "zweite adhäsive Schicht" bezeichnet. Die zweite adhäsive Schicht besteht aus einem Silikongel mit einer Klebkraft von 1,5 N/25 mm bis 2,7 N/25 mm wie nachfolgend noch genauer erläutert. Die wundabgewandte adhäsive Schicht der Trägerfolie wird vorliegend auch als "dritte adhäsive Schicht" bezeichnet. Die dritte adhäsive Schicht besteht ebenso wie die erste adhäsive Schicht aus einem Acrylatklebstoff. Die dritte adhäsive Schicht hat insbesondere die Funktion, die Wundkontaktschicht mit dem Wundkissen zu verbinden. Die Perforationen in der Trägerfolie der Wundkontaktschicht werden durch die zweite und dritte adhäsive Schicht nicht verschlossen. Die Perforationen durchdringen somit alle Schichten der Wundkontaktschicht, damit Wundexsudat in den Verband eindringen und ein Unterdruck an die Wundoberfläche weitergegeben werden kann.

Acrylatklebstoffe können eine hohe Haftkraft aufweisen und sind typischerweise kostengünstiger als Silikonklebstoffe. Indem der erfindungsgemäße Verband einen Rand mit einem Acrylatklebstoff aufweist, kann er folglich gut an der Wundstelle haften. Dadurch sind zusätzliche sekundäre Verbandsmaterialien zur Befestigung und Abdichtung bei dem vorliegenden Verband in der Regel nicht erforderlich. Durch die silikonisierte Wundkontaktschicht kann der erfindungsgemäße Verband wieder von der Wundstelle entfernt werden, ohne Schmerzen zu verursachen und die Wundoberfläche zu beschädigen. Zudem kann die Wundkontaktschicht mit dem Silikongel zur Haftung des Verbands an trockener, intakter Haut in der Wundumgebung beitragen. Der erfindungsgemäße Verband kann auch geringere Mengen des teuren Silikonklebstoffs enthalten, da die silikonisierte Wundkontaktschicht nur noch einen Teil der wundzugewandten Seite des Verbands bildet. Insgesamt kann mit dem vorliegenden Verband somit eine einfache, schnelle, kostengünstige und schonende Wundbehandlung durchgeführt werden. Weitere Vorteile des erfindungsgemäßen Verbands ergeben sich aus den nachfolgend angegebenen bevorzugten Ausführungsformen der Erfindung.

Die Begriffe wundzugewandt und wundabgewandt beziehen sich auf die bestimmungsgemäße Position des Verbands am Körper des Patienten. Eine wundzugewandte Seite einer Schicht des Verbands ist also diejenige Seite, welche bei bestimmungsgemäßem Gebrauch des Verbands der Wunde zugewandt ist. Üblicherweise handelt es sich bei der wundzugewandten Seite um die Unterseite. Entsprechend ist eine wundabgewandte Seite einer Schicht des Verbands diejenige Seite, welche bei bestimmungsgemäßem Gebrauch des Verbands der Wunde abgewandt ist. Üblicherweise handelt es sich bei der wundabgewandten Seite um die Oberseite.

Die Rückschicht ist die äußerste Schicht des Verbands. Sie grenzt den Verband nach außen ab, schützt die Wunde vor Kontamination und befestigt den Verband mit dem ersten Adhäsiv an der Wundstelle. Bei der Rückschicht kann es sich um eine geschlossene, durchgängige Schicht handeln. Wenn der Verband jedoch für eine Unterdruckwundtherapie vorgesehen ist, weist die Rückschicht vorteilhafterweise eine Öffnung zum Absaugen von Luft im Rahmen der Unterdruckwundtherapie auf. Durch die Öffnung kann der Verband in fluide Kommunikation mit einer Unterdruckquelle gebracht werden. Da die Rückschicht der Träger für die erste adhäsive Schicht ist, erstreckt sich die Öffnung in der Rückschicht auch auf die erste adhäsive Schicht. Das heißt, die Öffnung ist sowohl in der Rückschicht als auch in der ersten adhäsiven Schicht vorhanden. Die Öffnung kann zum Beispiel im Wesentlichen rund ausgestaltet sein und einen Durchmesser von 0,5 bis 2,5 cm aufweisen.

Die Rückschicht kann aus verschiedenen Materialien gebildet sein. Zum Beispiel kann die Rückschicht aus einem Vliesstoff oder einer flüssigkeitsundurchlässigen, wasserdampfdurchlässigen Kunststofffolie bestehen. Die Ausführungsform als Kunststofffolie ist insbesondere dann bevorzugt, wenn der Verband für eine Unterdruckwundtherapie vorgesehen ist. Die flüssigkeitsundurchlässige, wasserdampfdurchlässige Kunststofffolie kann zum Beispiel aus Polyurethan bestehen.

Bevorzugt ist die wundzugewandte Seite der Rückschicht vollflächig mit der ersten adhäsiven Schicht beschichtet. Das Wundkissen lässt sich dann leicht an der Rückschicht befestigen. Alternativ ist es jedoch auch möglich, dass die wundzugewandte Seite der Rückschicht nur im Bereich des ersten adhäsiven Rands mit dem ersten Adhäsiv beschichtet ist. Das Wundkissen kann in diesem Fall mit der Wundkontaktschicht im Verband eingeschlossen und gehalten werden. Dies wird im Folgenden im Zusammenhang mit dem zweiten adhäsiven Rand noch genauer beschrieben. Wenn nur der Rand der Rückschicht adhäsiv beschichtet ist, kann der Verband atmungsaktiver sein.

Die Klebkraft der ersten adhäsiven Schicht beträgt erfindungsgemäß mehr als 4 N/25 mm. Damit der Verband nicht zu stark an der Wundstelle haftet, beträgt die Klebkraft der ersten adhäsiven Schicht bevorzugt nicht mehr als 12 N/25 mm. Die Klebkraft der ersten adhäsiven Schicht wird nach DIN EN ISO 29862:2019 (Verfahren 1) bestimmt, wobei in folgenden Punkten von der Norm abgewichen wird:
- Als Substrat werden Polypropylen-Platten (PP-Platten) anstatt Stahlplatten verwendet. Die PP-Platten können Abmessungen von 150 mm x 50 mm x 4 mm aufweisen und als Einwegprüfkörper ausgebildet sein, deren Prüfseite mit Folie abgedeckt ist. Die Folie wird vor dem Test entfernt. Die PP-Platten können im Hinblick auf ihre Oberflächenbeschaffenheit den in der Norm vorgesehenen Stahlplatten entsprechen. Entsprechend sind die PP-Platten daher ebenso typischerweise vollständig eben ausgebildet und weisen eine glatte (Prüf)Oberfläche auf.
- Die Probenbreite ist 25 mm anstatt 24 mm.
- Die Wartezeit ist 20 min anstatt < 1 min (das heißt, die Probe wird erst 20 min nachdem sie auf die PP-Platte aufgebracht wurde vermessen).
- Gegebenenfalls wird ein Verlängerungstape verwendet, falls die Probe zu kurz ist (zum Beispiel das Tape Scotch 8959).
- Gegebenenfalls wird ein Verstärkungstape verwendet, falls die Probe elastisch ist (zum Beispiel das Tape Tesa 4104).

Für die Bestimmung der Klebkraft der ersten adhäsiven Schicht kann eine Zugprüfmaschine gemäß DIN EN ISO 7500-01:2018-06, Klasse 1 verwendet werden. Die Auswertung der Messergebnisse kann nach DIN ISO 6133:2017-04 (Verfahren B) erfolgen.

Zudem kann die Dicke der ersten adhäsiven Schicht 20 µm bis 60 µm betragen.

Wie für Inselverbände typisch dient der (erste) adhäsive Rand des Verbands dazu, den Verband an der Wundstelle zu befestigen. Der erste adhäsive Rand ist dafür vorgesehen ausschließlich an intakter Haut in der Wundumgebung befestigt zu werden. Entsprechend soll der erste adhäsive Rand die Wunde nicht berühren. Der erste adhäsive Rand kann beispielsweise eine Breite von 1,5 cm bis 5 cm aufweisen.

Erfindungsgemäß umfasst der vorliegende Verband weiterhin ein Wundkissen. Abhängig von dem Verwendungszweck des Verbands kann das Wundkissen sehr unterschiedlich ausgestaltet sein. Bevorzugte Merkmale und Varianten des Wundkissens sind in den nachfolgenden Ausführungsformen angegeben.

Typischerweise ist das Wundkissen im Wesentlichen zentral in Bezug auf die Rückschicht im Verband angeordnet. Zudem kann das Wundkissen einschichtig oder mehrschichtig ausgebildet sein. Insbesondere umfasst das Wundkissen eine oder mehrere Schichten ausgewählt aus der Gruppe bestehend aus einer absorbierenden Schicht, einer druckverteilenden Schicht und einer flüssigkeitsverteilenden Schicht. Nachfolgend werden die Funktion und bevorzugte Ausgestaltungen dieser drei verschiedenen Schichten beschrieben.

Eine absorbierende Schicht kann Flüssigkeit aufnehmen und speichern. Wenn das Wundkissen also eine absorbierende Schicht enthält, kann der Verband Wundflüssigkeit aufnehmen und speichern. Die absorbierende Schicht ist üblicherweise hydrophil ausgebildet. Sie kann zum Beispiel aus einem hydrophilen Vliesstoff oder einem hydrophilen Schaumstoff bestehen.

Bevorzugt umfasst der hydrophile Vliesstoff der absorbierenden Schicht Alginat-Fasern, zellulosebasierte Fasern und/oder ein superabsorbierendes Polymer (SAP) in Form von Fasern. Bei den zellulosebasierten Fasern kann es sich um Zellulosefasern oder Viskosefasern handeln. Bei dem superabsorbierenden Polymer kann es sich insbesondere um ein Polyacrylat handeln. Polyacrylate können sehr viel Flüssigkeit binden und sind daher besonders vorteilhafte Materialien für die absorbierende Schicht.

Bei dem hydrophilen Schaumstoff der absorbierenden Schicht handelt es sich bevorzugt um einen offenzelligen Schaumstoff. Vorzugsweise besteht der hydrophile Schaumstoff der absorbierenden Schicht aus Polyvinylalkohol oder Polyurethan.

In einer bevorzugten Ausführungsform der Erfindung besteht die absorbierende Schicht aus einem hydrophilen Vliesstoff, wobei der Vliesstoff zellulosebasierte Fasern, insbesondere Zellulosefasern oder Viskosefasern, und Polyacrylat-Fasern umfasst. Alternativ kann es ebenso bevorzugt sein, wenn die absorbierende Schicht aus einem hydrophilen, offenzelligen Schaumstoff aus Polyvinylalkohol oder Polyurethan besteht.

Die absorbierende Schicht kann auch im Hinblick auf ihre Absorptionskapazität weiter spezifiziert werden. So kann die absorbierende Schicht beispielsweise eine Absorptionskapazität von 50 g/100 cm² bis 300 g/100 cm², bevorzugt von 50 g/100 cm² bis 250 g/100 cm², mehr bevorzugt von 100 g/100 cm² bis 250 g/100 cm² und besonders bevorzugt von 140 g/100 cm² bis 220 g/100 cm² aufweisen. Dabei wird die Absorptionskapazität der absorbierenden Schicht nach DIN EN 13726-1:2002-06 (Kapitel 3.2) bestimmt.

Eine druckverteilende Schicht im Sinne der vorliegenden Erfindung kann einen Unterdruck im Verband verteilen. Luft und Flüssigkeit können aus der druckverteilenden Schicht abgesaugt werden, wenn die Schicht einem Unterdruck ausgesetzt wird. Wenn das Wundkissen also eine druckverteilende Schicht enthält, kann der Verband besonders gut für eine Unterdruckwundtherapie geeignet sein. Bevorzugt ist die druckverteilende Schicht hydrophob ausgebildet. Die druckverteilende Schicht kann zum Beispiel aus einem Abstandsgewirke, einem Gewebe, einer 3D-strukturierten Kunststofffolie oder einem hydrophoben, offenzelligen Schaumstoff bestehen.

Die 3D-strukturierte Kunststofffolie der druckverteilenden Schicht weist vorzugsweise eine Vielzahl von Perforationen auf, wobei zumindest ein Teil der Perforationen Erhebungen ausbilden. Die 3D-strukturierte Kunststofffolie der druckverteilenden Schicht kann insbesondere aus Polyethylen oder Polyurethan bestehen. Derartige Kunststofffolien mit einer dreidimensionalen Struktur und einer glatten sowie einer aufgerauten Seite sind aus der internationalen Patentanmeldung WO 2013/034263 A1 bekannt. Ihr Einsatz als druckverteilende Schicht ist auch in der deutschen Patentanmeldung mit der Anmeldenummer DE102022133930.0 beschrieben.

Der hydrophobe, offenzellige Schaumstoff der druckverteilenden Schicht besteht bevorzugt aus Polyurethan. In einer bevorzugten Ausführungsform der Erfindung besteht die druckverteilende Schicht demnach aus einem hydrophoben, offenzelligen Schaumstoff aus Polyurethan. Ein geeigneter hydrophober, offenzelliger Schaumstoff aus Polyurethan ist beispielsweise in der WO 2012/022485 A1 offenbart.

Vorteilhafterweise kann die druckverteilende Schicht eine Luftdurchlässigkeit von 1000 l/(m²sec) bis 8500 l/(m²sec), bevorzugt von 1500 l/(m²sec) bis 6000 l/(m²sec), mehr bevorzugt von 2000 l/(m²sec) bis 5000 l/(m²sec), besonders bevorzugt von 2300 l/(m²sec) bis 4000 l/(m²sec) und insbesondere von 2400 l/(m²sec) bis 3300 l/(m²sec) aufweisen. Dabei wird die Luftdurchlässigkeit der druckverteilenden Schicht nach DIN EN ISO 9237 (20 mm Prüfdicke, 20 cm² Prüffläche, 200 Pa Differenzdruck) bestimmt.

Eine flüssigkeitsverteilende Schicht kann entsprechend ihrer Bezeichnung Flüssigkeit verteilen. Die Flüssigkeit wird dabei insbesondere mittels Dochtwirkung in der Schicht verteilt. Wenn das Wundkissen eine flüssigkeitsverteilende Schicht enthält, kann Flüssigkeit wie Wundexsudat innerhalb des Wundkissens besser verteilt und die Absorptionskapazität einer in dem Wundkissen enthaltenen absorbierenden Schicht besser ausgenutzt werden. Es ist daher besonders vorteilhaft eine flüssigkeitsverteilende Schicht in dem Wundkissen vorzusehen, wenn das Wundkissen eine absorbierende Schicht umfasst. Die flüssigkeitsverteilende Schicht ist bevorzugt hydrophil ausgebildet. Sie kann zum Beispiel aus einem hydrophilen Vliesstoff bestehen. Der hydrophile Vliesstoff umfasst vorzugsweise zellulosebasierte Fasern, insbesondere Zellulosefasern oder Viskosefasern.

Es ist vorliegend vorgesehen, dass die flüssigkeitsverteilende Schicht zwar Flüssigkeit aufnehmen und verteilen, aber auch wieder leicht abgeben kann. Superabsorbierende Polymere können Flüssigkeiten jedoch sehr stark binden. Entsprechend umfasst die flüssigkeitsverteilende Schicht vorteilhafterweise kein superabsorbierendes Polymer, insbesondere kein Polyacrylat. Zudem weist die flüssigkeitsverteilende Schicht normalerweise eine geringere Absorptionskapazität als die absorbierende Schicht auf, wenn das Wundkissen sowohl eine absorbierende als auch eine flüssigkeitsverteilende Schicht umfasst.

Die flüssigkeitsverteilende Schicht kann eine Absorptionskapazität von 1 g/100 cm² bis 50 g/100 cm², bevorzugt von 1 g/100 cm² bis 40 g/100 cm², mehr bevorzugt von 1 g/100 cm² bis 25 g/100 cm², besonders bevorzugt von 1 g/100 cm² bis 10 g/100 cm² und insbesondere von 1 g/100 cm² bis 7 g/100 cm² aufweisen. Die Absorptionskapazität der flüssigkeitsverteilenden Schicht wird nach DIN EN 13726-1:2002-06 (Kapitel 3.2) bestimmt.

Im Hinblick auf die Anzahl und die Art der Schichten im Wundkissen werden die folgenden Varianten beispielhaft vorgeschlagen. In einer einfachen einschichtigen Ausführungsform der Erfindung besteht das Wundkissen aus einer absorbierenden Schicht. Der Verband kann sich dann insbesondere für eine klassische Wundbehandlung, also eine Wundbehandlung ohne den Einsatz von Unterdruck, bei gering bis mäßig stark exsudierenden Wunden eignen. Gemäß einer weiteren einfachen einschichtigen Ausgestaltung der Erfindung besteht das Wundkissen aus einer druckverteilenden Schicht. Der Verband kann sich dann insbesondere für eine Unterdruckwundtherapie eignen, wobei die Wundflüssigkeit in einem Behälter außerhalb des Verbands gesammelt wird und der Verband daher nicht zwingend eine absorbierende Schicht enthalten muss.

In einer vorteilhaften zweischichtigen Variante kann das Wundkissen aus einer absorbierenden Schicht und entweder einer druckverteilenden Schicht oder einer flüssigkeitsverteilenden Schicht bestehen. Dabei kann das Wundkissen derart im Verband angeordnet sein, dass die absorbierende Schicht an die Wundkontaktschicht und die druckverteilende oder flüssigkeitsverteilende Schicht an die Rückschicht beziehungsweise die erste adhäsive Schicht angrenzt. Vereinfacht ausgedrückt ist dann die druckverteilende oder flüssigkeitsverteilende Schicht über der absorbierenden Schicht im Verband angeordnet. Alternativ dazu kann das Wundkissen derart im Verband angeordnet sein, dass die absorbierende Schicht an die Rückschicht beziehungsweise die erste adhäsive Schicht und die druckverteilende oder flüssigkeitsverteilende Schicht an die Wundkontaktschicht angrenzt. Vereinfacht ausgedrückt ist dann die absorbierende Schicht über der druckverteilenden oder flüssigkeitsverteilenden Schicht im Verband angeordnet. Der Verband mit einer absorbierenden und druckverteilenden Schicht kann insbesondere für eine Unterdruckwundtherapie geeignet sein, bei der die Wundflüssigkeit im Verband verbleiben und eine kleine Unterdruckquelle ohne Sekretsammelbehälter verwendet werden soll. Der Verband mit einer absorbierenden und flüssigkeitsverteilenden Schicht kann insbesondere für die Behandlung von stark exsudierenden Wunden ohne den Einsatz von Unterdruck geeignet sein.

In einer besonders vorteilhaften dreischichtigen Version kann das Wundkissen aus einer absorbierenden Schicht und entweder zwei druckverteilenden Schichten oder zwei flüssigkeitsverteilenden Schichten bestehen. Dabei ist die absorbierende Schicht zwischen den beiden druckverteilenden oder flüssigkeitsverteilenden Schichten angeordnet. Ähnlich wie zuvor im Zusammenhang mit der zweischichtigen Ausführungsform beschrieben, ist die Kombination aus einer absorbierenden und zwei druckverteilenden Schichten insbesondere für die Unterdruckwundtherapie ohne Sekretsammelbehälter und die Kombination aus einer absorbierenden und zwei flüssigkeitsverteilenden Schichten insbesondere für die Behandlung von stark exsudierenden Wunden ohne Unterdruck vorgesehen. Im Vergleich zu der zweischichtigen Variante des Wundkissens kann die vorliegende dreischichte Ausführungsform den Unterdruck beziehungsweise die Flüssigkeit jedoch noch besser im Verband verteilen.

Wenn das Wundkissen mehrschichtig aufgebaut ist, können die Schichten im Wesentlichen gleich groß ausgebildet und aufeinandergelegt sein. Dabei können die Schichten vollflächig miteinander verbunden sein, so dass das Wundkissen in der Art eines Laminats ausgebildet ist. Es kann jedoch auch vorteilhaft sein, wenn das Wundkissen taschenartig ausgestaltet ist. Entsprechend wird eine Ausführungsform vorgeschlagen, in der das Wundkissen eine Hülle und einen in der Hülle befindlichen absorbierenden Kern umfasst. Die Hülle kann aus zwei Vliesstoff-Schichten bestehen, wobei die Vliesstoff-Schichten in ihren Randbereichen miteinander verbunden sind und dadurch eine Tasche ausbilden, in welcher der absorbierende Kern aufgenommen ist. Bevorzugt sind die Vliesstoffschichten mit einer Ultraschallschweißnaht verbunden. Der absorbierende Kern umfasst insbesondere Zellstoff und ein superabsorbierendes Polymer in Form von Partikeln, insbesondere Polyacrylat-Partikel. Da der absorbierende Kern umhüllt ist, können die vorzugsweise lose im Zellstoff eingebrachten SAP-Partikel nicht aus dem Wundkissen austreten. Insofern stellt die Hülle den strukturellen Zusammenhalt des Wundkissens sicher. Eine oder beide der zuvor genannten Vliesstoffschichten der Hülle können bevorzugt auch eine flüssigkeitsverteilende Schicht im Sinne der vorliegenden Erfindung darstellen.

Im Folgenden wird näher auf die silikonisierte Wundkontaktschicht des vorliegenden Verbands eingegangen. Die Wundkontaktschicht ist typischerweise im Wesentlichen zentral in Bezug auf die Rückschicht im Verband angeordnet. Weiterhin ist die Wundkontaktschicht üblicherweise im Wesentlichen mindestens so groß wie das Wundkissen ausgebildet, damit sie die wundzugewandte Seite des Wundkissens vollständig bedecken kann.

In einer Ausführungsform der Erfindung sind die Wundkontaktschicht und das Wundkissen im Wesentlichen gleich groß ausgebildet und schließen im Wesentlichen bündig ab. Das Wundkissen mit der Wundkontaktschicht kann dann mit der ersten adhäsiven Schicht an der Rückschicht befestigt werden. Diese Ausführungsform kann einfach hergestellt werden und ist kostengünstig.

Alternativ kann die Wundkontaktschicht das Wundkissen überragen, so dass die Wundkontaktschicht einen adhäsiven Rand aufweist. Der adhäsive Rand der Wundkontaktschicht wird vorliegend auch als "zweiter adhäsiver Rand" bezeichnet. Der zweite adhäsive Rand ist zumindest teilweise mit der Rückschicht beziehungsweise der ersten adhäsiven Schicht verbunden, so dass die Rückschicht und die Wundkontaktschicht eine Tasche zur Aufnahme des Wundkissens ausbilden können. Bei dieser Ausführungsform kann die Wundkontaktschicht das Wundkissen im Verband halten. Es ist dann möglich, die Rückschicht nur in ihrem Randbereich adhäsiv zu beschichten, um den Verband atmungsaktiver auszugestalten. Außerdem ist es dann möglich, dass das Wundkissen aus mehreren, lediglich lose übereinandergelegten Schichten gebildet ist. Damit kann ein hinsichtlich seiner Zusammensetzung sehr variables mehrschichtiges Wundkissen auf einfache Art und Weise bereitgestellt werden. Die lediglich lose übereinandergelegten Schichten des Wundkissens können sich nicht voneinander trennen, weil die Rückschicht und die Wundkontaktschicht das Wundkissen umschließen.

Ferner kann die Wundkontaktschicht 30 % bis 90 %, bevorzugt 30 % bis 80 %, mehr bevorzugt 30 % bis 70 % und besonders bevorzugt 50 % bis 70 % einer wundzugewandten Seite des Verbands bilden. Die durch die Perforationen erhaltene offene Fläche in der Wundkontaktschicht kann dabei unberücksichtigt bleiben. Weiterhin kann die Trägerfolie der Wundkontaktschicht aus Polyurethan bestehen. Die Dicke der Trägerfolie der Wundkontaktschicht kann beispielhaft etwa 25 µm betragen.

Silikongele für Wundauflagen sind den Fachmann ebenso wie Acrylatklebstoffe bekannt und zum Beispiel in der EP 4 218 698 A1 näher beschrieben. Das Silikongel der Wundkontaktschicht hat eine geringere Klebkraft als der an der Rückschicht angeordnete Acrylatklebstoff. Entsprechend beträgt die Klebkraft der zweiten adhäsiven Schicht erfindungsgemäß nur 1,5 N/25 mm bis 2,7 N/25 mm. Die Klebkraft der zweiten adhäsiven Schicht wird nach FINAT Nr. 1 (FTM 1 Peel adhesion (180°) at 300 mm per minute) bestimmt, wobei in folgenden Punkten von der Norm abgewichen wird:
- Als Substrat wird Bristolpapier anstatt Glas verwendet. Bei dem Bristolpapier kann es sich zum Beispiel um Oxford, unifarbenes weißes Papier, nicht perforiert, Referenz 237000 handeln.
- Die Kontroll-Atmosphäre ist 20 ± 2 °C, 60 ± 5 % rF oder 23 ± 2 °C, 50 ± 5 % rF.
- Die Messungen werden innerhalb von 20 Minuten nach der Probenherstellung durchgeführt.
Die Dicke der zweiten adhäsiven Schicht kann 80 µm bis 200 µm, vorzugsweise 100 µm bis 150 µm, betragen.

Der Acrylatklebstoff der Wundkontaktschicht (anspruchsgemäße dritte adhäsive Schicht) kann bezüglich seiner Klebkraft und Dicke im Wesentlichen genauso ausgebildet sein wie der an der Rückschicht angeordnete Acrylatklebstoff (anspruchsgemäße erste adhäsive Schicht). Die dritte adhäsive Schicht kann jedoch auch eine andere Klebkraft und Dicke als die erste adhäsive Schicht haben. Vorzugsweise beträgt die Klebkraft der dritten adhäsiven Schicht 2 N/25 mm bis 7 N/25 mm, insbesondere etwa 4,5 N/25 mm. Die Klebkraft der dritten adhäsiven Schicht kann nach FINAT Nr. 1 (FTM 1 Peel adhesion (90°) at 300 mm per minute) bestimmt werden, wobei in folgenden Punkten von der Norm abgewichen werden kann:
- Als Substrat wird eine Stahlplatte anstatt Glas verwendet. Die Stahlplatte kann eine Abmessung von 50 mm x 150 mm (Breite x Länge) aufweisen und ist typischerweise vollständig eben und glatt ausgebildet.
- Die Kontroll-Atmosphäre ist 20 ± 2 °C, 60 ± 5 % rF oder 23 ± 2 °C, 50 ± 5 % rF.
- Die Messungen werden unmittelbar nach der Vorbereitung der Probenstücke durchgeführt.
Die Dicke der dritten adhäsiven Schicht kann 30 µm bis 50 µm betragen. Jedenfalls sind die Klebkraft und Dicke der dritten adhäsiven Schicht typischerweise so zu wählen, dass die Wundkontaktschicht dauerhaft an dem Wundkissen und gegebenenfalls auch an der Rückschicht befestigt werden kann.

Die perforierte Wundkontaktschicht kann ein Flächengewicht von 150 g/m² bis 200 g/m² aufweisen. Dabei können 70 g/m² bis 130 g/m² auf das Silikongel entfallen.

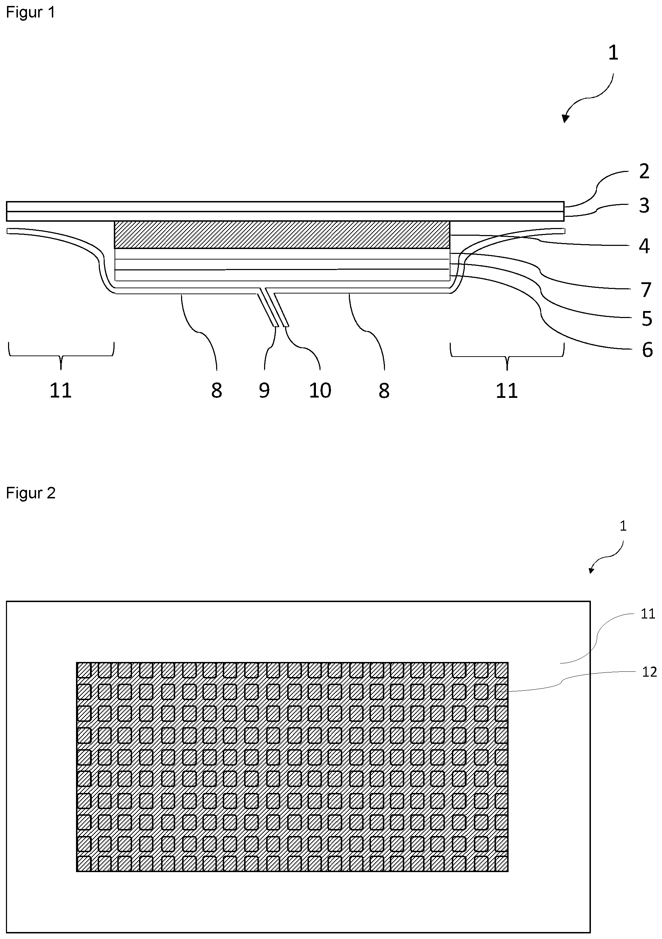

Wie bereits erläutert ist der vorliegende Verband insbesondere für die Unterdruckwundtherapie vorgesehen. Dabei kann es vorteilhaft sein, wenn der Verband weiterhin einen Saugschlauch zum Verbinden mit einer Unterdruckquelle im Rahmen einer Unterdruckwundtherapie umfasst. Der Saugschlauch weist bevorzugt an einem wundseitigen Ende ein Anschlussstück auf, welches derart ausgebildet ist, dass das wundseitige Ende des Saugschlauchs an einer wundabgewandten Seite der Rückschicht luftdicht befestigt werden kann. Dabei wird das Anschlussstück in der Regel oberhalb einer Öffnung in der Rückschicht befestigt, damit der Saugschlauch in fluider Kommunikation mit dem Wundkissen des Verbands stehen kann. Anschlussstücke für Verbände in der Unterdruckwundtherapie, auch Ports genannt, sind dem Fachmann bekannt. Ein solcher Port ist zum Beispiel in der WO 2011/076340 A1 oder der WO 2017/097834 A1 offenbart. Darüber hinaus kann der Saugschlauch vorteilhafterweise an einem wundfernen Ende einen Teil eines Steckverbinders beziehungsweise Konnektors aufweisen, um leicht an einer Unterdruckquelle oder einem Sekretsammelbehälter angeschlossen werden zu können. Ein derartiger Konnektor mit Verschluss ist in Figur 1a der WO 2017/097834 A1 im linken Bildrand beispielhaft gezeigt.

Zudem kann es bei Einsatz des Verbands in der Unterdruckwundtherapie sinnvoll sein, wenn der Verband weiterhin einen flüssigkeitsundurchlässigen, luftdurchlässigen Filter umfasst. Der Filter kann die zuvor genannte Öffnung in der Rückschicht flüssigkeitsdicht verschließen, so dass keine Wundflüssigkeit aus dem Verband abgesaugt werden kann. Diese Ausführungsform ist insbesondere für einfach aufgebaute und kleine Unterdruckwundtherapiesysteme gedacht, bei denen die Wundflüssigkeit im Verband von einer absorbierenden Schicht aufgenommen wird und die Unterdruckquelle keinen Sekretsammelbehälter aufweist.

Je nach Ausführungsform des Verbands kann der Filter an verschiedenen Stellen im Verband angeordnet sein. Der Filter ist bevorzugt direkt mit der Rückschicht verbunden und verschließt dabei die zuvor genannte Öffnung in der Rückschicht. Wenn der Verband das vorangehend beschriebene Anschlussstück umfasst, kann der Filter alternativ auch in dem Anschlussstück angeordnet sein. Vorzugsweise handelt es sich bei dem Filter um eine Filtermembran.

Der Verband umfasst außer der ersten und zweiten adhäsiven Schicht vorzugsweise keine weitere mit einer Haut- oder Wundoberfläche eines Patienten in Kontakt tretende adhäsive Schicht.

Wie bereits eingangs deutlich gemacht wurde ist der Verband insbesondere dafür vorgesehen beziehungsweise ausgebildet ohne einen separat bereitgestellten Klebestreifen zum Befestigen und/oder Abdichten des Verbands am Körper eines Patienten für die Wundbehandlung verwendet zu werden. Dabei wird auf solche Klebestreifen abgestellt, die dafür vorgesehen sind an einer Hautoberfläche eines Patienten und einer wundabgewandten Seite der Rückschicht befestigt zu werden. Normalerweise bestehen solche Klebestreifen aus einer einseitig adhäsiv beschichteten Kunststofffolie und optional einer Abziehfolie als Applikationssystem.

Besonders bevorzugt ist der Verband dafür vorgesehen beziehungsweise ausgebildet ohne ein separat bereitgestelltes Mittel zum Befestigen und/oder Abdichten des Verbands am Körper eines Patienten für die Wundbehandlung verwendet zu werden. Jegliche zusätzliche Befestigungs- und Abdichtmittel sind damit bei dieser bevorzugten Ausführungsform von der Verwendung in Verbindung mit dem Verband ausgenommen. Beispielsweise wären dann neben den zuvor genannten Klebestreifen auch flüssige, in Tuben bereitgestellte Abdichtmittel oder die aus der EP 0 782 421 B1 bekannten Hydrogelstreifen ausgenommen, welche zwischen der Haut und der Rückschicht zum Ausgleichen von Unebenheiten angeordnet werden können.

Der Verband umfasst erfindungsgemäß zumindest die Rückschicht, die erste adhäsive Schicht, das Wundkissen sowie die dreilagige, perforierte Wundkontaktschicht. Weitere Komponenten des Verbands können der bereits beschriebene Saugschlauch mitsamt Anschlussstück und Steckverbinder, der Filter sowie eine Abziehfolie sein. Entsprechend kann der vorliegende Verband aus den folgenden Komponenten bestehen:
i. die Rückschicht
ii. die erste adhäsive Schicht
iii. das Wundkissen
iv. die Wundkontaktschicht bestehend aus der perforierten Trägerfolie, der zweiten adhäsiven Schicht und der dritten adhäsiven Schicht
v. optional der Abziehfolie
vi. optional dem Filter
vii. optional dem Saugschlauch, vorzugsweise dem Saugschlauch mit Anschlussstück und/oder Steckverbinder
Die Abziehfolie bedeckt die klebende wundzugewandte Seite des Verbands. Sie erleichtert die Handhabung des Verbands und schützt den Verband vor Kontamination während der Lagerung. Die Abziehfolie wird vom Anwender entfernt, bevor der Verband an der Wundstelle befestigt wird. Die Abziehfolie kann mehrteilig, zum Beispiel zweiteilig, ausgebildet sein. Applikationssysteme für selbstklebende Wundauflagen mit einer solchen Abziehfolie sind üblich und dem Fachmann bekannt.

Gegenstand der vorliegenden Erfindung ist auch ein Kit für eine Unterdruckwundtherapie. Der Kit umfasst einen Verband wie vorangehend beschrieben und einen Saugschlauch zum Verbinden des Verbands mit einer Unterdruckquelle. Der Saugschlauch kann in dem Kit getrennt von dem Verband vorliegen. Der Saugschlauch ist dann herstellerseitig noch nicht am Verband befestigt. Zudem kann der Saugschlauch in dem Kit wie bereits erwähnt ein Anschlussstück und/oder einen Steckverbinder umfassen. Der Kit ist typischerweise steril und kann weiterhin zum Beispiel eine Gebrauchsanweisung enthalten. Der Kit umfasst jedoch vorzugsweise keinen separat von dem Verband bereitgestellten Klebestreifen zum Befestigen und/oder Abdichten des Verbands am Körper eines Patienten, insbesondere kein separat von dem Verband bereitgestelltes Mittel zum Befestigen und/oder Abdichten des Verbands am Körper eines Patienten. Auch hierbei sind wieder nur solche Klebestreifen gemeint, die dafür vorgesehen sind an einer Hautoberfläche eines Patienten und einer wundabgewandten Seite der Rückschicht befestigt zu werden.

Ein weiterer Gegenstand der vorliegenden Erfindung ist ein System für eine Unterdruckwundtherapie. Das System umfasst einen Verband wie vorangehend beschrieben, eine Unterdruckquelle und einen Saugschlauch zum Verbinden des Verbands mit der Unterdruckquelle.

Schließlich betrifft die Erfindung auch ein Verfahren für eine Wundbehandlung umfassend die Schritte:
i. Bereitstellen eines Verbands, Kits oder Systems wie vorangehend beschrieben.
ii. Befestigen des Verbands an einer Wundstelle, wobei über die Dauer der gesamten Wundbehandlung vorzugsweise kein separat bereitgestellter Klebestreifen zum Befestigen und/oder Abdichten des Verbands, insbesondere kein separat bereitgestelltes Mittel zum Befestigen und/oder Abdichten des Verbands, verwendet wird.

Anders ausgedrückt betrifft die Erfindung somit auch den Verband, den Kit oder das System wie vorangehend beschrieben zur Anwendung in der Behandlung einer Wunde, insbesondere zur Anwendung in der Behandlung einer Wunde mit Unterdruck, wobei über die Dauer der gesamten Wundbehandlung vorzugsweise kein separat bereitgestellter Klebestreifen zum Befestigen und/oder Abdichten des Verbands, insbesondere kein separat bereitgestelltes Mittel zum Befestigen und/oder Abdichten des Verbands, verwendet wird.

Der Verband kann in all seinen vorangehend beschriebenen Ausführungsformen für den Kit, das System und das Verfahren verwendet werden. Die zusätzlichen Merkmale des Verbands in seinen speziellen Ausführungsformen sind daher ebenso im Zusammenhang mit dem Kit, dem System sowie dem Verfahren offenbart.

### Figuren

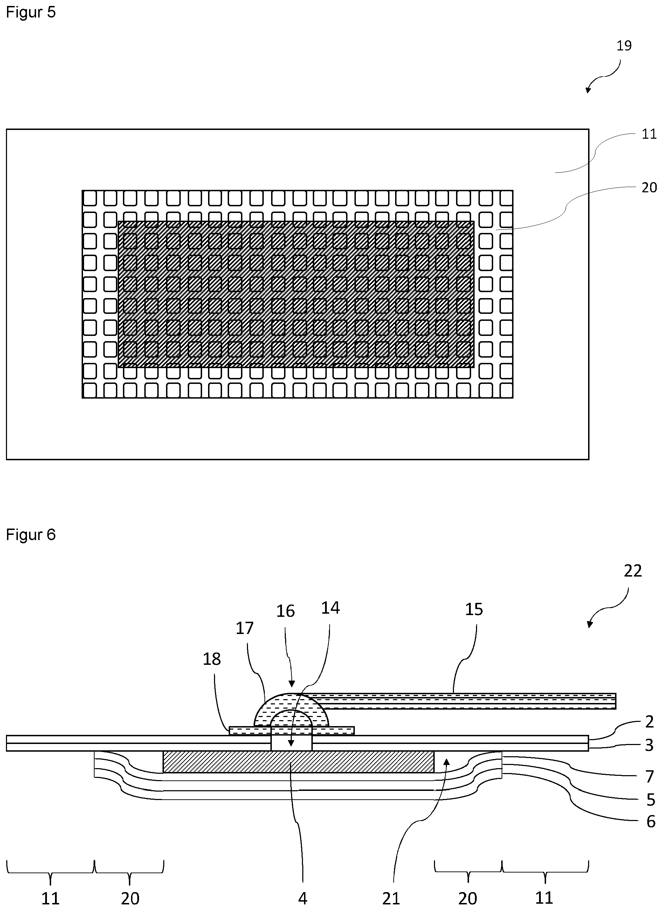

Mit den nachfolgend beschriebenen, schematischen Figuren soll die Erfindung exemplarisch näher erläutert und veranschaulicht werden. Dabei können in den Figuren ähnliche Strukturelemente mit denselben Bezugszeichen ausgewiesen sein. Die Verbände sind außer in den Figuren 2 und 5 jeweils in einer seitlichen Schnittansicht dargestellt.

**Figur 1** zeigt einen erfindungsgemäßen Verband **1** in einer ersten Ausführungsform. Der Verband **1** umfasst eine Rückschicht **2.** Bei der Rückschicht **2** handelt es sich bevorzugt um eine transparente, beispielsweise aus Polyurethan bestehende flüssigkeitsundurchlässige und wasserdampfdurchlässige Kunststofffolie. Die Rückschicht **2** ist an ihrer wundzugewandten Unterseite vollflächig mit einem Acrylatklebstoff **3** beschichtet. Der Acrylatklebstoff **3** bildet die anspruchsgemäße erste adhäsive Schicht des Verbands 1. Weiterhin umfasst der Verband **1** ein Wundkissen **4.** Dieses besteht hier aus einer einzigen Schicht, bei der es sich bevorzugt um eine absorbierende Schicht handelt. Das Wundkissen **4** und die Rückschicht **2** werden mit dem Acrylatklebstoff **3** verbunden. Außerdem umfasst der Verband **1** eine dreischichtige, perforierte Wundkontaktschicht, die ebenso wie die adhäsive Rückschicht **2, 3** transparent sein kann. Die Wundkontaktschicht besteht aus einer Trägerfolie **5,** welche beidseitig adhäsiv beschichtet ist. Auf ihrer wundzugewandten Unterseite ist die Trägerfolie **5** mit einem Silikongel **6** (anspruchsgemäße zweite adhäsive Schicht) beschichtet. Auf ihrer wundabgewandten Oberseite ist die Trägerfolie **5** mit einem Acrylatklebstoff **7** (anspruchsgemäße dritte adhäsive Schicht) beschichtet. Mit dem Acrylatklebstoff **7** kann die Wundkontaktschicht auf einfache Weise an dem Wundkissen **4** befestigt werden. Die in Figur 1 nicht dargestellten Perforationen in der Wundkontaktschicht durchdringen alle Schichten der Wundkontaktschicht gleichermaßen. Sie können beispielsweise in die Wundkontaktschicht geschnitten oder gestanzt sein. Schließlich umfasst der Verband **1** auch noch eine zweiteilige Abziehfolie **8** mit Grifflaschen **9, 10.** Die Abziehfolie **8** schützt die adhäsive Unterseite des Verbands **1** und wird entfernt, bevor der Verband **1** an der Wundstelle befestigt wird.

Wie in Figur 1 dargestellt überragt die adhäsive Rückschicht **2, 3** sowohl das Wundkissen **4** als auch die Wundkontaktschicht **5, 6, 7,** so dass der Verband **1** einen adhäsiven Rand **11** (anspruchsgemäßer erster adhäsiver Rand) aufweist. Mit dem adhäsiven Rand **11** wird der Verband 1 an der Wundstelle befestigt. Die Wundkontaktschicht **5, 6, 7** und das Wundkissen **4** sind hinsichtlich ihrer flächenhaften Ausdehnung gleich groß ausgebildet und schließen an ihren Rändern bündig ab. Durch die zentrale Positionierung des Verbunds aus Wundkissen **4** und Wundkontaktschicht **5, 6, 7** auf der adhäsiven Rückschicht **2, 3** ergibt sich die für einen Inselverband typische Ausgestaltung.

**Figur 2** zeigt die Unterseite des Verbands **1** aus Figur 1 (ohne die Abziehfolie **8).** Zu erkennen sind nun die Perforationen **12** in der Wundkontaktschicht **5, 6, 7.** Sie sind in regelmäßigen Abständen über die gesamte Fläche der Wundkontaktschicht verteilt angeordnet. Dabei ist die Anzahl, Größe und Form der Perforationen **12** beispielhaft zu verstehen. Insbesondere können die Perforationen **12** auch rund ausgebildet sein. Durch die erfindungsgemäße Kombination aus stark haftendem Acrylatklebstoff 3 im Randbereich **11** und schwach haftendem Silikongel 6 im zentralen, wundkontaktierenden Bereich kann der Verband 1 einerseits fest an der Wundstelle haften und andererseits von der Wundstelle wieder entfernt werden, ohne dabei die Wundoberfläche zu beschädigen.

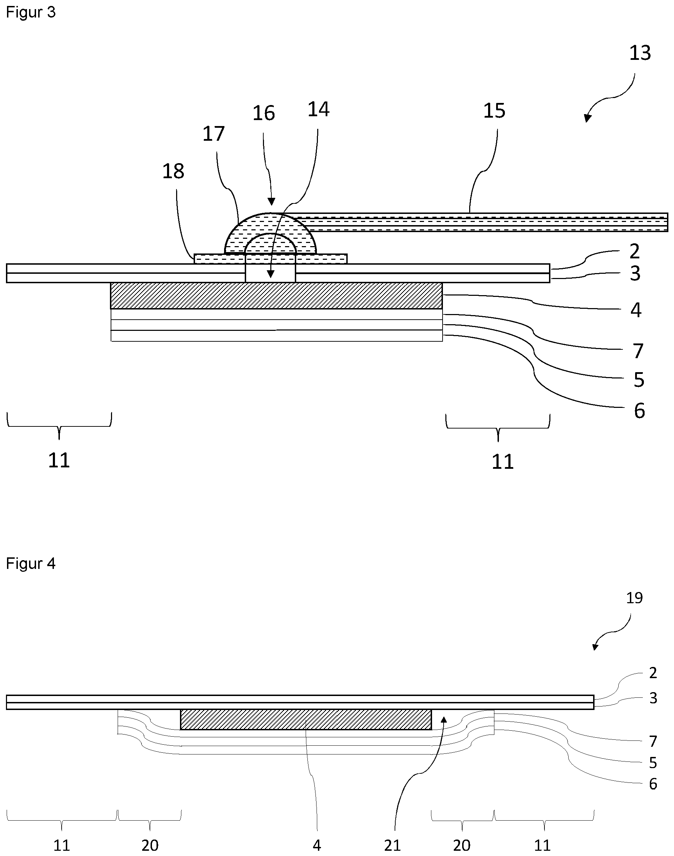

**Figur 3** zeigt einen erfindungsgemäßen Verband **13** in einer zweiten Ausführungsform. Der Verband **13** ist ähnlich aufgebaut wie der Verband **1** aus den Figuren 1 und 2. Im Unterschied zu der vorangehend gezeigten Variante weist der Verband **13** jedoch eine Öffnung **14** in der adhäsiven Rückschicht **2,** 3 auf. An der nicht-adhäsiven, wundabgewandten Außen- beziehungsweise Oberseite der Rückschicht **2** ist ein Saugschlauch **15** mit einem Anschlussstück **16** direkt über der Öffnung **14** befestigt. Das Anschlussstück **16** umfasst im dargestellten Fall ein hohles, kuppelförmiges Gehäuse **17** mit einem adhäsiv ausgebildeten Flansch **18,** mit dem es an der Rückschicht **2** luft- und flüssigkeitsdicht befestigt werden kann. Der Saugschlauch **15** ist wiederum an dem Gehäuse **17** fluidleitend befestigt. Der Saugschlauch **15** mitsamt Anschlussstück **16** besteht typischerweise überwiegend aus einem im Wesentlichen transparenten, flexiblen Kunststoff, zum Beispiel Silikon oder Polyvinylchlorid (PVC). Mit dem Saugschlauch **15** kann der Verband **13** mit einer Unterdruckquelle (nicht dargestellt) verbunden und dann einem Unterdruck ausgesetzt werden.

Bei dem Wundkissen **4** kann es sich bei dem Verband **13** anstatt um eine absorbierende Schicht auch vorteilhafterweise um eine druckverteilende Schicht wie bereits zuvor erläutert handeln. Der Verband **13** kann auch eine Abziehfolie zum Schutz seiner klebenden Unterseite aufweisen. Zur Vereinfachung wurde die Abziehfolie jedoch in dieser Figur und ebenso in den nachfolgenden Figuren weggelassen.

In **Figur 4** ist eine dritte Ausführungsform der Erfindung als Verband **19** dargestellt. Der Verband **19** unterscheidet sich von dem Verband **1** aus den Figuren 1 und 2 dadurch, dass die Wundkontaktschicht **5, 6, 7** das Wundkissen **4** überragt. Die Wundkontaktschicht **5, 6, 7** weist somit einen adhäsiven Rand **20** auf (anspruchsgemäßer zweiter adhäsiver Rand), welcher zumindest teilweise mit der adhäsiven Rückschicht **2, 3** verbunden ist. Die adhäsive Rückschicht **2, 3** und die Wundkontaktschicht **5, 6,** 7 bilden dann eine Tasche **21** zur Aufnahme des Wundkissens **4** aus. In dieser Ausführungsform kann das Wundkissen **4** besonders gut im Verband fixiert werden. Der Verband **19** mit Blick auf seine Unterseite ist in **Figur 5** gezeigt.

**Figur 6** zeigt einen erfindungsgemäßen Verband **22** in einer vierten Ausführungsform. Der Verband **22** basiert auf dem Verband **19** aus den Figuren 4 und 5. Zusätzlich weist er jedoch die bereits aus Figur 3 bekannte Öffnung **14** und den Saugschlauch mit Anschlussstück **15, 16** auf, damit der Verband **22** für eine Unterdruckwundtherapie verwendet werden kann.

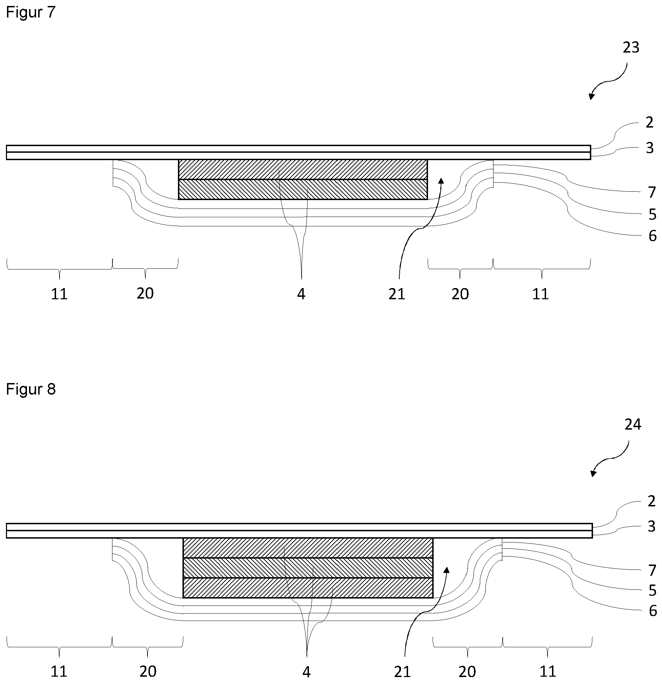

In **Figur 7** ist mit dem Bezugszeichen **23** eine fünfte Ausführungsform eines erfindungsgemäßen Verbands gezeigt. Der Verband **23** basiert gleichfalls auf dem Verband **19** aus den Figuren 4 und 5. Anders als zuvor ist das Wundkissen **4** hier allerdings mehrschichtig, nämlich zweischichtig, aufgebaut. Zum Beispiel könnte die obere, an die adhäsive Rückschicht **2, 3** angrenzende Schicht des Wundkissens 4 eine absorbierende Schicht sein. Die untere, an die Wundkontaktschicht **5, 6, 7** angrenzende Schicht könnte eine flüssigkeitsverteilende Schicht sein. Mit diesem Aufbau kann die Absorptionskapazität der absorbierenden Schicht besser ausgenutzt werden, da die flüssigkeitsverteilende Schicht das durch die Wundkontaktschicht **5, 6, 7** hindurchtretende Wundexsudat über die gesamte Oberfläche der absorbierenden Schicht verteilen kann.

Schließlich ist in **Figur 8** ein erfindungsgemäßer Verband **24** als sechste Ausführungsform gezeigt. Diese entspricht der zuvor beschriebenen Variante von Figur 7 mit der Ausnahme, dass das Wundkissen **4** aus drei Schichten besteht. Vorteilhaft wäre es zum Beispiel, wenn die mittlere Schicht des Wundkissens eine absorbierende Schicht ist und die beiden äußeren Schichten des Wundkissens **4** flüssigkeitsverteilende Schichten sind. Die absorbierende Schicht ist dann zwischen den beiden flüssigkeitsverteilenden Schichten im Wundkissen **4** aufgenommen. Dies führt zu einer noch besseren Verteilung des Wundexsudats im Wundkissen 4.

Die in den Figuren 1 bis 8 gezeigten Verbände haben alle eine rechteckige Form. Die Verbände können jedoch auch rund, oval oder herzförmig ausgestaltet sein. Die herzförmige Ausführung kann beispielsweise für die Behandlung von Wunden im Sakralbereich geeignet sein. Ebenso ist es vorgesehen, dass die erfindungsgemäßen Verbände nicht nur in verschiedenen Formen, sondern auch in verschiedenen Größen zur Verfügung gestellt werden. Verbände in unterschiedlichen Formen und Größen sind gängige Praxis in der Wundversorgung, damit möglichst für jede Wunde unabhängig davon wo sie sich am Körper des Patienten befindet und unabhängig von ihrer Größe ein passender Verband verfügbar ist und ausgewählt werden kann.

## Patentansprüche

1. Verband (1, 13, 19, 22, 23, 24) für eine Wundbehandlung, umfassend
- eine Rückschicht (2),
- eine erste adhäsive Schicht (3), welche an einer wundzugewandten Seite der Rückschicht (2) angeordnet ist,
- ein Wundkissen (4),
- eine Wundkontaktschicht (5, 6, 7),
wobei das Wundkissen (4) zwischen der Rückschicht (2) und der Wundkontaktschicht (5, 6, 7) angeordnet ist, und
wobei die Rückschicht (2) mit der ersten adhäsiven Schicht (3) sowohl das Wundkissen (4) als auch die Wundkontaktschicht (5, 6, 7) überragt, so dass der Verband einen ersten adhäsiven Rand (11) bestehend aus der Rückschicht (2) und der ersten adhäsiven Schicht (3) aufweist,
**dadurch gekennzeichnet, dass**
- die erste adhäsive Schicht (3) aus einem Acrylatklebstoff besteht und die Klebkraft der ersten adhäsiven Schicht (3) mehr als 4 N/25 mm beträgt, bestimmt nach DIN EN ISO 29862:2019 wie im vorliegenden Dokument beschrieben,
- die Wundkontaktschicht aus einer perforierten Trägerfolie (5), einer zweiten adhäsiven Schicht (6) und einer dritten adhäsiven Schicht (7) besteht,
- wobei die zweite adhäsive Schicht (6) an einer wundzugewandten Seite der Trägerfolie (5) angeordnet ist und aus einem Silikongel besteht, wobei die Klebkraft der zweiten adhäsiven Schicht (6) 1,5 N/25 mm bis 2,7 N/25 mm beträgt, bestimmt nach FINAT Nr. 1 wie im vorliegenden Dokument beschrieben, und
- wobei die dritte adhäsive Schicht (7) an einer wundabgewandten Seite der Trägerfolie (5) angeordnet ist und aus einem Acrylatklebstoff besteht.

2. Verband nach Anspruch 1, **dadurch gekennzeichnet, dass** die Rückschicht (2) eine Öffnung (14) zum Absaugen von Luft im Rahmen einer Unterdruckwundtherapie aufweist.

3. Verband nach Anspruch 1 oder 2, **dadurch gekennzeichnet, dass** das Wundkissen (4) eine oder mehrere Schichten ausgewählt aus der Gruppe bestehend aus einer absorbierenden Schicht, einer druckverteilenden Schicht und einer flüssigkeitsverteilenden Schicht umfasst.

4. Verband nach Anspruch 3, **dadurch gekennzeichnet, dass** die absorbierende Schicht aus einem hydrophilen Vliesstoff oder einem hydrophilen Schaumstoff besteht.

5. Verband nach Anspruch 4, **dadurch gekennzeichnet, dass** der hydrophile Vliesstoff Alginat-Fasern, zellulosebasierte Fasern und/oder ein superabsorbierendes Polymer in Form von Fasern umfasst.

6. Verband nach einem der Ansprüche 3 bis 5, **dadurch gekennzeichnet, dass**
- die absorbierende Schicht aus einem hydrophilen Vliesstoff besteht, wobei der Vliesstoff zellulosebasierte Fasern, insbesondere Zellulosefasern oder Viskosefasern, und Polyacrylat-Fasern umfasst, oder
- die absorbierende Schicht aus einem hydrophilen, offenzelligen Schaumstoff aus Polyvinylalkohol oder Polyurethan besteht.

7. Verband nach einem der Ansprüche 3 bis 6, **dadurch gekennzeichnet, dass** die absorbierende Schicht eine Absorptionskapazität von 50 g/100 cm² bis 300 g/100 cm², bevorzugt von 50 g/100 cm² bis 250 g/100 cm², mehr bevorzugt von 100 g/100 cm² bis 250 g/100 cm² und besonders bevorzugt von 140 g/100 cm² bis 220 g/100 cm² aufweist, bestimmt nach DIN EN 13726-1:2002-06 (Kapitel 3.2).

8. Verband nach einem der Ansprüche 3 bis 7, **dadurch gekennzeichnet, dass** die druckverteilende Schicht aus einem Abstandsgewirke, einem Gewebe, einer 3D-strukturierten Kunststofffolie oder einem hydrophoben, offenzelligen Schaumstoff besteht.

9. Verband nach einem der Ansprüche 3 bis 8, **dadurch gekennzeichnet, dass** die druckverteilende Schicht eine Luftdurchlässigkeit von 1000 l/(m²sec) bis 8500 l/(m²sec), bevorzugt von 1500 l/(m²sec) bis 6000 l/(m²sec), mehr bevorzugt von 2000 l/(m²sec) bis 5000 l/(m²sec), besonders bevorzugt von 2300 l/(m²sec) bis 4000 l/(m²sec) und insbesondere von 2400 l/(m²sec) bis 3300 l/(m²sec) aufweist, bestimmt nach DIN EN ISO 9237.

10. Verband nach einem der Ansprüche 3 bis 9, **dadurch gekennzeichnet, dass** die flüssigkeitsverteilende Schicht aus einem hydrophilen Vliesstoff besteht, wobei der hydrophile Vliesstoff vorzugsweise zellulosebasierte Fasern, insbesondere Zellulosefasern oder Viskosefasern, umfasst.

11. Verband nach einem der Ansprüche 3 bis 10, **dadurch gekennzeichnet, dass** die flüssigkeitsverteilende Schicht kein superabsorbierendes Polymer umfasst und/oder eine geringere Absorptionskapazität aufweist als die absorbierende Schicht.

12. Verband nach einem der Ansprüche 3 bis 11, **dadurch gekennzeichnet, dass** die flüssigkeitsverteilende Schicht eine Absorptionskapazität von 1 g/100 cm² bis 50 g/100 cm², bevorzugt von 1 g/100 cm² bis 40 g/100 cm², mehr bevorzugt von 1 g/100 cm² bis 25 g/100 cm², besonders bevorzugt von 1 g/100 cm² bis 10 g/100 cm² und insbesondere von 1 g/100 cm² bis 7 g/100 cm² aufweist, bestimmt nach DIN EN 13726-1:2002-06 (Kapitel 3.2).

13. Verband nach einem der Ansprüche 3 bis 9, **dadurch gekennzeichnet, dass** das Wundkissen (4) aus einer absorbierenden Schicht oder einer druckverteilenden Schicht besteht.

14. Verband nach einem der Ansprüche 3 bis 12, **dadurch gekennzeichnet, dass** das Wundkissen (4) aus einer absorbierenden Schicht und entweder einer druckverteilenden Schicht oder einer flüssigkeitsverteilenden Schicht besteht, wobei das Wundkissen (4) derart im Verband angeordnet ist, dass entweder
- die absorbierende Schicht an die Wundkontaktschicht (5, 6, 7) und die druckverteilende oder flüssigkeitsverteilende Schicht an die Rückschicht (2) angrenzt oder
- die absorbierende Schicht an die Rückschicht (2) und die druckverteilende oder flüssigkeitsverteilende Schicht an die Wundkontaktschicht (5, 6, 7) angrenzt.

15. Verband nach einem der Ansprüche 3 bis 12, **dadurch gekennzeichnet, dass** das Wundkissen (4) aus einer absorbierenden Schicht und entweder zwei druckverteilenden Schichten oder zwei flüssigkeitsverteilenden Schichten besteht, wobei die absorbierende Schicht zwischen den beiden druckverteilenden oder flüssigkeitsverteilenden Schichten angeordnet ist.

16. Verband nach einem der vorangehenden Ansprüche, **dadurch gekennzeichnet, dass**
- die Wundkontaktschicht (5, 6, 7) und das Wundkissen (4) im Wesentlichen gleich groß ausgebildet sind und im Wesentlichen bündig abschließen, oder
- die Wundkontaktschicht (5, 6, 7) das Wundkissen (4) überragt, so dass die Wundkontaktschicht (5, 6, 7) einen zweiten adhäsiven Rand (20) aufweist, wobei der zweite adhäsive Rand (20) zumindest teilweise mit der Rückschicht (2) verbunden ist, so dass die Rückschicht (2) und die Wundkontaktschicht (5, 6, 7) eine Tasche (21) zur Aufnahme des Wundkissens (4) ausbilden können.

17. Verband nach einem der vorangehenden Ansprüche, **dadurch gekennzeichnet, dass** die Wundkontaktschicht (5, 6, 7) 30 % bis 90 %, bevorzugt 30 % bis 80 %, mehr bevorzugt 30 % bis 70 % und besonders bevorzugt 50 % bis 70 % einer wundzugewandten Seite des Verbands bildet.

18. Verband nach einem der vorangehenden Ansprüche, **dadurch gekennzeichnet, dass** der Verband außer der ersten und zweiten adhäsiven Schicht (3, 6) keine weitere mit einer Haut- oder Wundoberfläche eines Patienten in Kontakt tretende adhäsive Schicht umfasst.

19. Verband nach einem der vorangehenden Ansprüche, **dadurch gekennzeichnet, dass** der Verband dafür vorgesehen ist ohne einen separat bereitgestellten Klebestreifen zum Befestigen und/oder Abdichten des Verbands am Körper eines Patienten, insbesondere ohne ein separat bereitgestelltes Mittel zum Befestigen und/oder Abdichten des Verbands am Körper eines Patienten, für die Wundbehandlung verwendet zu werden.

20. Kit für eine Unterdruckwundtherapie, umfassend
- einen Verband nach einem der vorangehenden Ansprüche,
- einen Saugschlauch (15) zum Verbinden des Verbands mit einer Unterdruckquelle, und
- optional eine Gebrauchsanweisung,
wobei der Kit vorzugsweise keinen Klebestreifen zum Befestigen und/oder Abdichten des Verbands am Körper eines Patienten, insbesondere kein Mittel zum Befestigen und/oder Abdichten des Verbands am Körper eines Patienten, umfasst.

21. System für eine Unterdruckwundtherapie, umfassend
- einen Verband nach einem der Ansprüche 1 bis 19,
- eine Unterdruckquelle, und
- einen Saugschlauch (15) zum Verbinden des Verbands mit der Unterdruckquelle.

22. Verband, Kit oder System nach einem der vorangehenden Ansprüche zur Anwendung in der Behandlung einer Wunde, wobei über die Dauer der gesamten Wundbehandlung vorzugsweise kein separat bereitgestellter Klebestreifen zum Befestigen und/oder Abdichten des Verbands, insbesondere kein separat bereitgestelltes Mittel zum Befestigen und/oder Abdichten des Verbands, verwendet wird.

## Claims

1. Dressing (1, 13, 19, 22, 23, 24) for wound treatment, comprising
- a backing layer (2),
- a first adhesive layer (3), disposed on a wound-facing side of the backing layer (2),
- a wound pad (4),
- a wound contact layer (5, 6, 7),
wherein the wound pad (4) is disposed between the backing layer (2) and the wound contact layer (5, 6, 7), and
wherein the backing layer (2) with the first adhesive layer (3) projects beyond both the wound pad (4) and the wound contact layer (5, 6, 7), and so the dressing has a first adhesive margin (11) consisting of the backing layer (2) and the first adhesive layer (3),
**characterized in that**
- the first adhesive layer (3) consists of an acrylate adhesive and the peel adhesion of the first adhesive layer (3) is more than 4 N/25 mm, determined according to DIN EN ISO 29862:2019 as described in the present document,
- the wound contact layer consists of a perforated carrier film (5), a second adhesive layer (6) and a third adhesive layer (7),
- wherein the second adhesive layer (6) is disposed on a wound-facing side of the carrier film (5) and consists of a silicone gel, wherein the peel adhesion of the second adhesive layer (6) is 1.5 N/25 mm to 2.7 N/25 mm, determined according to FINAT No. 1 as described in the present document, and
- wherein the third adhesive layer (7) is disposed on a wound-remote side of the carrier film (5) and consists of an acrylate adhesive.

2. Dressing according to Claim 1, **characterized in that** the backing layer (2) has an opening (14) for drawing off air as part of a negative-pressure wound therapy.

3. Dressing according to Claim 1 or 2, **characterized in that** the wound pad (4) comprises one or more layers selected from the group consisting of an absorbent layer, a pressure distribution layer and a fluid distribution layer.

4. Dressing according to Claim 3, **characterized in that** the absorbent layer consists of a hydrophilic nonwoven or a hydrophilic foam.

5. Dressing according to Claim 4, **characterized in that** the hydrophilic nonwoven comprises alginate fibres, cellulose-based fibres and/or a superabsorbent polymer in the form of fibres.

6. Dressing according to any of Claims 3 to 5, **characterized in that**
- the absorbent layer consists of a hydrophilic nonwoven, wherein the nonwoven comprises cellulose-based fibres, more particularly cellulose fibres or viscose fibres, and polyacrylate fibres, or
- the absorbent layer consists of a hydrophilic, open-cell foam of polyvinyl alcohol or polyurethane.

7. Dressing according to any of Claims 3 to 6, **characterized in that** the absorbent layer has an absorption capacity of 50 g/100 cm² to 300 g/100 cm², preferably of 50 g/100 cm² to 250 g/100 cm², more preferably of 100 g/100 cm² to 250 g/100 cm² and particularly preferably of 140 g/100 cm² to 220 g/100 cm², determined according to DIN EN 13726-1:2002-06 (section 3.2).

8. Dressing according to any of Claims 3 to 7, **characterized in that** the pressure distribution layer consists of a spacer knit, a woven fabric, a 3D-structured plastics film or a hydrophobic, open-cell foam.

9. Dressing according to any of Claims 3 to 8, **characterized in that** the pressure distribution layer has an air permeability of 1000 l/(m²sec) to 8500 l/(m²sec), preferably of 1500 l/(m²sec) to 6000 l/(m²sec), more preferably of 2000 l/(m²sec) to 5000 l/(m²sec), particularly preferably of 2300 l/(m²sec) to 4000 l/(m²sec) and more particularly of 2400 l/(m²sec) to 3300 l/(m²sec), determined according to DIN EN ISO 9237.

10. Dressing according to any of Claims 3 to 9, **characterized in that** the fluid distribution layer consists of a hydrophilic nonwoven, wherein the hydrophilic nonwoven comprises preferably cellulose-based fibres, more particularly cellulose fibres or viscose fibres.

11. Dressing according to any of Claims 3 to 10, **characterized in that** the fluid distribution layer comprises no superabsorbent polymer and/or has a lower absorption capacity than the absorbent layer.

12. Dressing according to any of Claims 3 to 11, **characterized in that** the fluid distribution layer has an absorption capacity of 1 g/100 cm² to 50 g/100 cm², preferably of 1 g/100 cm² to 40 g/100 cm², more preferably of 1 g/100 cm² to 25 g/100 cm², particularly preferably of 1 g/100 cm² to 10 g/100 cm² and more particularly of 1 g/100 cm² to 7 g/100 cm², determined according to DIN EN 13726-1:2002-06 (section 3.2).

13. Dressing according to any of Claims 3 to 9, **characterized in that** the wound pad (4) consists of an absorbent layer or a pressure distribution layer.

14. Dressing according to any of Claims 3 to 12, **characterized in that** the wound pad (4) consists of an absorbent layer and either a pressure distribution layer or a fluid distribution layer, wherein the wound pad (4) is disposed in the dressing in such a way that either
- the absorbent layer borders the wound contact layer (5, 6, 7) and the pressure distribution or fluid distribution layer borders the backing layer (2), or
- the absorbent layer borders the backing layer (2) and the pressure distribution or fluid distribution layer borders the wound contact layer (5, 6, 7).

15. Dressing according to any of Claims 3 to 12, **characterized in that** the wound pad (4) consists of an absorbent layer and either two pressure distribution layers or two fluid distribution layers, wherein the absorbent layer is disposed between the two pressure distribution or fluid distribution layers.

16. Dressing according to any of the preceding claims, **characterized in that**
- the wound contact layer (5, 6, 7) and the wound pad (4) are of substantially equal size and finish substantially flush, or
- the wound contact layer (5, 6, 7) projects beyond the wound pad (4), and so the wound contact layer (5, 6, 7) has a second adhesive margin (20), wherein the second adhesive margin (20) is connected at least partly to the backing layer (2), and so the backing layer (2) and the wound contact layer (5, 6, 7) can form a pocket (21) for accommodating the wound pad (4).

17. Dressing according to any of the preceding claims, **characterized in that** the wound contact layer (5, 6, 7) forms 30% to 90%, preferably 30% to 80%, more preferably 30% to 70% and particularly preferably 50% to 70% of a wound-facing side of the dressing.

18. Dressing according to any of the preceding claims, **characterized in that** other than the first and second adhesive layers (3, 6), the dressing comprises no further adhesive layer entering into contact with a skin surface or wound surface of a patient.

19. Dressing according to any of the preceding claims, **characterized in that** the dressing is envisaged to be used without a separately provided adhesive strip for securing and/or sealing the dressing on the body of a patient, more particularly without a separately provided means for securing and/or sealing the dressing on the body of a patient, for wound treatment.

20. Kit for a negative-pressure wound therapy, comprising
- a dressing according to any of the preceding claims,
- a suction hose (15) for connecting the dressing to a negative-pressure source, and
- optionally a set of use instructions,
wherein the kit preferably comprises no adhesive strip for securing and/or sealing the dressing on the body of a patient, more particularly no means for securing and/or sealing the dressing on the body of a patient.

21. System for a negative-pressure wound therapy, comprising
- a dressing according to any of Claims 1 to 19,
- a negative-pressure source, and
- a suction hose (15) for connecting the dressing to the negative-pressure source.

22. Dressing, kit or system according to any of the preceding claims for usage in the treatment of a wound, wherein over the duration of the entire wound treatment, preferably no separately provided adhesive strip for securing and/or sealing the dressing, more particularly no separately provided means for securing and/or sealing the dressing, is used.

## Revendications

1. Bandage (1, 13, 19, 22, 23, 24) pour un traitement de plaie, comprenant :
- une couche arrière (2),
- une première couche adhésive (3), qui est disposée sur un côté de la couche arrière (2) tourné vers la plaie,
- un coussinet pour plaie (4),
- une couche de contact avec la plaie (5, 6, 7),
dans lequel le coussinet pour plaie (4) est disposé entre la couche arrière (2) et la couche de contact avec la plaie (5, 6, 7), et
dans lequel la couche arrière (2) avec la première couche adhésive (3) dépasse à la fois le coussinet pour plaie (4) et la couche de contact avec la plaie (5, 6, 7), de sorte que le bandage présente un premier bord adhésif (11) constitué de la couche arrière (2) et de la première couche adhésive (3),
**caractérisé en ce que**
- la première couche adhésive (3) est constituée d'un adhésif acrylate et la force adhésive de la première couche adhésive (3) est supérieure à 4 N/25 mm, déterminée selon la norme DIN EN ISO 29862:2019 tel que décrit dans le présent document,
- la couche de contact avec la plaie est constituée d'un film support perforé (5), d'une deuxième couche adhésive (6) et d'une troisième couche adhésive (7),
- la deuxième couche adhésive (6) étant disposée sur un côté du film support (5) tourné vers la plaie et est constituée d'un gel de silicone, la force adhésive de la deuxième couche adhésive (6) étant de 1,5 N/25 mm à 2,7 N/25 mm, déterminée selon FINAT n° 1 tel que décrit dans le présent document, et
- la troisième couche adhésive (7) étant disposée sur un côté du film support (5) opposé à la plaie et étant constituée d'un adhésif acrylate.

2. Bandage selon la revendication 1, **caractérisé en ce que** la couche arrière (2) présente une ouverture (14) destinée à l'aspiration d'air dans le cadre d'une thérapie des plaies par pression négative.

3. Bandage selon la revendication 1 ou 2, **caractérisé en ce que** le coussinet pour plaie (4) comprend une ou plusieurs couches sélectionnées dans le groupe constitué d'une couche absorbante, d'une couche de répartition de pression et d'une couche de répartition de liquide.

4. Bandage selon la revendication 3, **caractérisé en ce que** la couche absorbante est constituée d'un non-tissé hydrophile ou d'une mousse hydrophile.

5. Bandage selon la revendication 4, **caractérisé en ce que** le non-tissé hydrophile comprend des fibres d'alginate, des fibres à base de cellulose et/ou un polymère superabsorbant sous forme de fibres.

6. Bandage selon l'une quelconque des revendications 3 à 5, **caractérisé en ce que**
- la couche absorbante est constituée d'un non-tissé hydrophile, le non-tissé comprenant des fibres à base de cellulose, notamment des fibres de cellulose ou des fibres de viscose, et des fibres de polyacrylate, ou
- la couche absorbante est constituée d'une mousse hydrophile à cellules ouvertes en alcool polyvinylique ou en polyuréthane.

7. Bandage selon l'une quelconque des revendications 3 à 6, **caractérisé en ce que** la couche absorbante présente une capacité d'absorption de 50 g/100 cm² à 300 g/100 cm², de préférence de 50 g/100 cm² à 250 g/100 cm², plus préférablement de 100 g/100 cm² à 250 g/100 cm² et de manière particulièrement préférée de 140 g/100 cm² à 220 g/100 cm², déterminée selon la norme DIN EN 13726-1:2002-06 (chapitre 3.2).

8. Bandage selon l'une quelconque des revendications 3 à 7, **caractérisé en ce que** la couche de répartition de pression est constituée d'un tricot d'espacement, d'un tissu, d'un film plastique à structure 3D ou d'une mousse hydrophobe à cellules ouvertes.

9. Bandage selon l'une quelconque des revendications 3 à 8, **caractérisé en ce que** la couche de répartition de pression présente une perméabilité à l'air de 1000 l/(m²sec) à 8500 l/(m²sec), de préférence de 1500 l/(m²sec) à 6000 l/(m²sec), plus préférablement de 2000 l/(m²sec) à 5000 l/(m²sec), de manière particulièrement préférée de 2300 l/(m²sec) à 4000 l/(m²sec) et notamment de 2400 l/(m²sec) à 3300 l/(m²sec), déterminée selon la norme DIN EN ISO 9237.

10. Bandage selon l'une quelconque des revendications 3 à 9, **caractérisé en ce que** la couche de répartition de liquide est constituée d'un non-tissé hydrophile, le non-tissé hydrophile comprenant de préférence des fibres à base de cellulose, notamment des fibres de cellulose ou des fibres de viscose.

11. Bandage selon l'une quelconque des revendications 3 à 10, **caractérisé en ce que** la couche de répartition de liquide ne comprend pas de polymère superabsorbant et/ou présente une capacité d'absorption inférieure à celle de la couche absorbante.

12. Bandage selon l'une quelconque des revendications 3 à 11, **caractérisé en ce que** la couche de répartition de liquide présente une capacité d'absorption de 1 g/100 cm² à 50 g/100 cm², de préférence de 1 g/100 cm² à 40 g/100 cm², plus préférablement de 1 g/100 cm² à 25 g/100 cm², de manière particulièrement préférée de 1 g/100 cm² à 10 g/100 cm² et notamment de 1 g/100 cm² à 7 g/100 cm², déterminée selon la norme DIN EN 13726-1:2002-06 (chapitre 3.2).

13. Bandage selon l'une quelconque des revendications 3 à 9, **caractérisé en ce que** le coussinet pour plaie (4) est constitué d'une couche absorbante ou d'une couche de répartition de pression.

14. Bandage selon l'une quelconque des revendications 3 à 12, **caractérisé en ce que** le coussinet pour plaie (4) est constitué d'une couche absorbante et soit d'une couche de répartition de pression, soit d'une couche de répartition de liquide, le coussinet pour plaie (4) étant disposé dans le bandage de telle sorte que soit
- la couche absorbante est adjacente à la couche de contact avec la plaie (5, 6, 7) et la couche de répartition de pression ou la couche de répartition de liquide est adjacente à la couche arrière (2), soit
- la couche absorbante est adjacente à la couche arrière (2) et la couche de répartition de pression ou la couche de répartition de liquide est adjacente à la couche de contact avec la plaie (5, 6, 7).

15. Bandage selon l'une quelconque des revendications 3 à 12, **caractérisé en ce que** le coussinet pour plaie (4) est constitué d'une couche absorbante et soit de deux couches de répartition de pression, soit de deux couches de répartition de liquide, la couche absorbante étant disposée entre les deux couches de répartition de pression ou de répartition de liquide.

16. Bandage selon l'une quelconque des revendications précédentes, **caractérisé en ce que**
- la couche de contact avec la plaie (5, 6, 7) et le coussinet pour plaie (4) sont réalisés essentiellement de même taille et affleurent essentiellement l'un avec l'autre, ou
- la couche de contact avec la plaie (5, 6, 7) dépasse le coussinet pour plaie (4), de sorte que la couche de contact avec la plaie (5, 6, 7) présente un deuxième bord adhésif (20), le deuxième bord adhésif (20) étant relié au moins partiellement à la couche arrière (2), de sorte que la couche arrière (2) et la couche de contact avec la plaie (5, 6, 7) peuvent former une poche (21) destinée à recevoir le coussinet pour plaie (4).

17. Bandage selon l'une quelconque des revendications précédentes, **caractérisé en ce que** la couche de contact avec la plaie (5, 6, 7) forme 30 % à 90 %, de préférence 30 % à 80 %, plus préférablement 30 % à 70 % et de manière particulièrement préférée 50 % à 70 % d'un côté du bandage tourné vers la plaie.

18. Bandage selon l'une quelconque des revendications précédentes, **caractérisé en ce que** le bandage ne comprend, en plus de la première et de la deuxième couche adhésive (3, 6), aucune autre couche adhésive entrant en contact avec une surface cutanée ou une surface de plaie d'un patient.

19. Bandage selon l'une quelconque des revendications précédentes, **caractérisé en ce que** le bandage est prévu pour être utilisé pour le traitement de plaie sans bande adhésive fournie séparément pour fixer et/ou étanchéifier le bandage sur le corps d'un patient, notamment sans moyen fourni séparément pour fixer et/ou étanchéifier le bandage sur le corps d'un patient.

20. Kit pour une thérapie des plaies par pression négative, comprenant :
- un bandage selon l'une quelconque des revendications précédentes,
- un tuyau d'aspiration (15) destiné à relier le bandage à une source de pression négative, et
- éventuellement une notice d'utilisation,
le kit ne comprenant de préférence aucune bande adhésive destinée à fixer et/ou à étanchéifier le bandage sur le corps d'un patient, notamment aucun moyen destiné à fixer et/ou à étanchéifier le bandage sur le corps d'un patient.

21. Système pour une thérapie des plaies par pression négative, comprenant :
- un bandage selon l'une quelconque des revendications 1 à 19,
- une source de pression négative, et
- un tuyau d'aspiration (15) destiné à relier le bandage à la source de pression négative.

22. Bandage, kit ou système selon l'une quelconque des revendications précédentes destiné à être utilisé dans le traitement d'une plaie, aucune bande adhésive fournie séparément pour fixer et/ou étanchéifier le bandage, notamment aucun moyen fourni séparément pour fixer et/ou étanchéifier le bandage, n'étant de préférence utilisé pendant toute la durée du traitement de la plaie.
